# EUROPÄISCHE PATENTANMELDUNG

(11) **EP 1 873 136 A1**
(43) Veröffentlichungstag der Anmeldung: **02.01.2008**
(21) Anmeldenummer: 07010660.4
(22) Anmeldetag: 30.05.2007
(51) Int. Cl.: C07C 41/01, C07D 213/64, C07D 333/32, C07C 43/205

(54) **Verfahren zur Herstellung von Alkyl- und Arylethern**

(30) Priorität: 07.06.2006 DE 102006026431
(71) Anmelder: Archimica GmbH, 65929 Frankfurt am Main (DE)
(72) Erfinder: Meudt, Andreas, Dr., 65719 Hofheim (DE); Rausch, Bernhard J., Dr., 65812 Bad Soden (DE)
(74) Vertreter: Schweitzer, Klaus

(57) **Zusammenfassung**

Die Erfindung betrifft ein Verfahren zur Herstellung von organischen Verbindungen durch Erzeugung von Alkoholatverbindungen aus Alkoholen und Base oder Einsatz käuflicher Alkoholate und deren Umsetzung mit geeigneten Arylhalogeniden in der Gegenwart von Kupfersalzen und geeigneten Liganden (Gleichung 1).

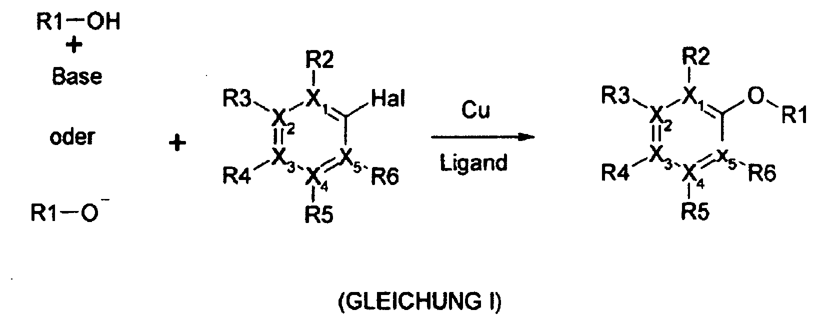

## Beschreibung

Die Erfindung betrifft ein Verfahren zur Herstellung von organischen Verbindungen durch Erzeugung von Alkoholatverbindungen aus Alkoholen und Base oder Einsatz käuflicher Alkoholate und deren Umsetzung mit geeigneten Arylhalogeniden in der Gegenwart von Kupfersalzen und geeigneten Liganden (Gleichung I).

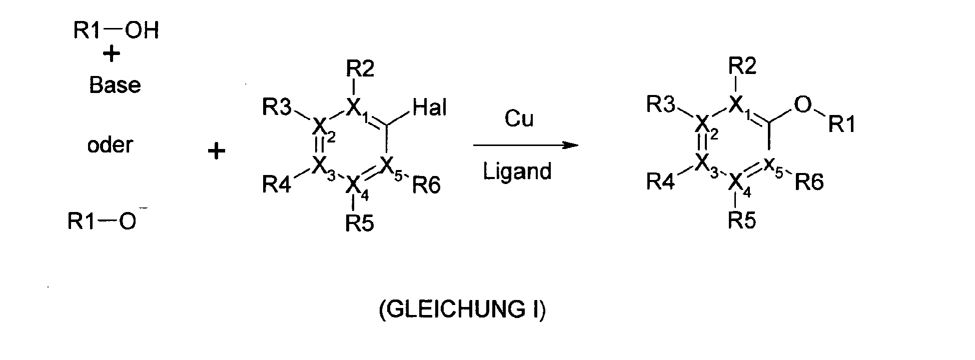

Die Kupfervermittelten Kupplungsreaktionen haben im Zuge des allgemeinen Aufschwungs der metallorganischen Chemie in den letzten Jahren stark an Bedeutung gewonnen. Solche Synthesemethoden spielen insbesondere bei der Herstellung von Verbindungen für die pharmazeutische und agrochemische Industrie eine entscheidende Rolle, da sich dadurch die immer komplexer werdenden Strukturen der benötigten Feinchemikalien für die Bereiche Pharma und Agro aufbauen lassen.

Dabei bieten die Kupfervermittelten C,X-Verknüpfungsreaktionen (X = Heteroatom), wie beispielsweise Kohlenstoff-Stickstoff-Kupplungen, Kohlenstoff-Sauerstoff-Kupplungen oder Kohlenstoff-Schwefel-Kupplungen ein sehr vielfältiges Synthesepotential für den Aufbau komplexer organischer Strukturen.

Eine Vielzahl von Kupplungsreaktionen ist bekannt, wobei die meisten Verknüpfungsreaktionen durch folgende Charakteristika gekennzeichnet werden:
(1) Bei Durchführung der Reaktion werden Liganden zugesetzt, die mit dem Kupfermetall einen Komplex bilden. Die Liganden sind dabei aromatische oder aliphatische Amine oder Alkohole.
(2) Die Reaktionen erreichen Umsätze (bestimmt über GC oder HPLC) von typischerweise 30 bis 80 %, meist zwischen 50 und 70 %. Somit verbleiben größere Mengen der Edukte (Arylhalogenide) in den Reaktionsmischungen, welche durch aufwendige Aufarbeitungsmethoden entfernt werden müssen.
(3) Typische Reaktionszeiten liegen bei 24 bis 48 h, meist zwischen 36 und 42 h.
(4) Nach Durchführung der Synthese wird die Reaktionsmischung typischerweise einer wässrigen Aufarbeitung unterzogen, wobei oftmals die organischen Liganden gemeinsam mit dem Kupplungsprodukt in der organischen Phase vorliegen, was häufig eine aufwendige Aufarbeitungsmethode, wie z.B. Säulenchromatographie, notwendig macht.
(5) Die Reaktionen werden verdünnt in organischen Lösemitteln durchgeführt,
wobei bevorzugt Lösemittel wie 2-Propanol, Toluol, Ethylenglykol, Methanol, 1,4-Dioxan, 1,2-Dimethoxyethan, Triethylamin bzw. deren Mischungen verwendet werden.

Eine Beispiel für eine Kupfervermittelte C,O-Verknüpfungsreaktion wird von Buchwald et al. beschrieben (WO 02/085 838 A1). Bei diesem Zugang wird beispielsweise 1-Butoxy-3,5-dimethylbenzol aus 3,5-Dimethyliodbenzol und n-Butanol in der Gegenwart von Cul, Cs₂CO₃ und eines Liganden gebildet (Gleichung II). Typische Liganden sind dabei 2-Phenylphenol, 2,6-Dimethylphenol, 2-Isopropylphenol, 1-Naphthol, N,N-Dimethylglycin, Methyliminodiessigsäure und N,N,N',N'-Tetramethylethylendiamin. Dabei werden Ausbeuten von 20 bis 81 % erreicht bei einer Reaktionszeit von 36 h bei 105°C.

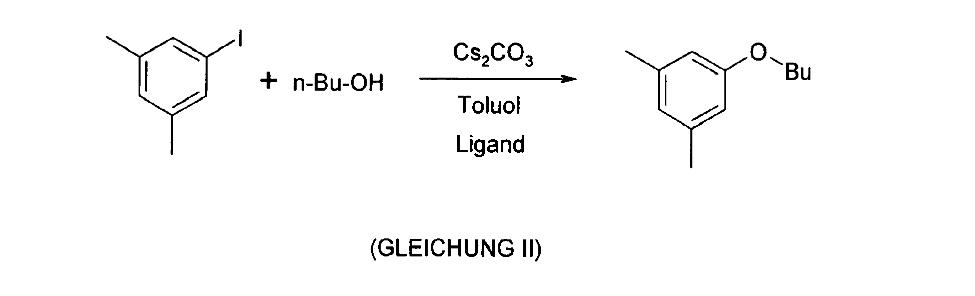

Die besten Ausbeuten werden dabei mit 2-Phenylphenol (= 2-Hydroxybiphenyl) erreicht, einer Verbindung, die als sehr umwelttoxisch und als potentiell krebserregend (R 40) eingestuft ist. Dies stellt einen erheblichen Nachteil dieses Verfahrens dar und macht den Einsatz dieses Prozesses - über die allgemeinen Probleme beim technischen Einsatz solcher CMR-Stoffe in der Produktion hinaus - für die Herstellung von Pharma-Feinchemikalien sehr schwierig.

Des Weiteren ist ein Nachteil des Verfahrens, dass nach dem Abbruch der Reaktion durch wässrige Aufarbeitung die organischen Produktphasen mit dem Liganden verunreinigt werden. Die häufig gut in klassischen organischen Lösemitteln löslichen Liganden bedingen, dass diese nur durch aufwendige Zusatzoperationen von den Kupplungsprodukten getrennt werden können. Außerdem enthalten auf Grund der hohen Affinität des Metalls zu diesen Liganden die Produkte oftmals Reste an Kupfer. Dies ist ebenfalls aus produktionstechnischer Sicht nachteilig und unwirtschaftlich.

In einigen Fällen werden große Mengen an teilweise komplexen Lösungsmittelgemischen zur Durchführung der Verfahren eingesetzt, welche für die industrielle Produktion jedoch einen erheblichen Kostenfaktor darstellen. Die Lösungsmittelgemische müssten über aufwendige, energieintensive Destillationsverfahren getrennt werden, so dass häufig deren Auftrennung nicht ökonomisch durchführbar ist. Für eine industrielle Großproduktion ist allerdings die Rückführung der verwendeten Lösungsmittel unabdingbare Voraussetzung.

Die beschriebenen Reaktionen werden typischerweise bei Eduktkonzentrationen von ca. 2 bis 5 % durchgeführt. Aus technischer Sicht ist dieses verdünnte Verfahren unwirtschaftlich, da eine derart niedrige Raum-Zeitausbeute bei der Durchführung von Produktionsprozessen zu hohen Herstellkosten führen würde.

Die von Buchwald et al. beschriebenen C,O-Verknüpfungen starten typischerweise von Aryliodidverbindungen, um Umsätze von 60 bis 80 % in der Kupplungsreaktion erreichen zu können, jedoch ist aus wirtschaftlicher Sicht der Einsatz der günstigeren Bromide und Chloride zu bevorzugen.

Um Feinchemikalien für Anwendungen im Pharmabereich einsetzen zu können, müssen diese oftmals strenge Kriterien bezüglich der chemischen Reinheit und des Isomerengehalts erfüllen. Um dieses Ziel zu erreichen ist ein effektives Katalysatorsystem notwendig, das vollständige Umsätze der eingesetzten Arylhalogenide in die entsprechenden C,O-Kupplungsprodukte ermöglicht, denn oftmals sind Eduktreste schlecht oder nur unter großem Aufwand aus dem Kupplungsprodukt zu entfernen. Die von Buchwald beschrieben Methode der C,O-Verknüpfung liefert lediglich Umsätze von typischerweise 60-80%, was die Darstellung hochreiner, eduktfreier Produkte mit diesem Verfahren stark erschwert und teilweise sogar unmöglich macht.

Aus den genannten Gründen wäre es daher sehr wünschenswert, ein Verfahren zu haben, das unter Einsatz geringer Mengen nicht toxischer oder umweltgefährlicher Liganden eine Kupferkatalysierte C,O-Kupplung mit einem möglichst quantitativen Umsatz ermöglicht, wobei die Liganden bei der Aufarbeitung leicht vom organischen Kupplungsprodukt abzutrennen sein sollten. Dabei wäre es insbesondere auch wünschenswert, ein deutlich aktiveres Kupfer-Ligand-Katalysatorsystem als die bisher beschriebenen Systeme zur Verfügung zu haben, das es ermöglicht, die Reaktionszeiten von derzeit typischerweise 36-42 h zu verringern und die Raum-Zeit-Ausbeuten durch Durchführung der Reaktion bei Eduktkonzentrationen von > 10 % deutlich zu verbessern. Des Weiteren sollte ein solches Verfahren kupferfreie Produkte dadurch liefern, dass die einzusetzenden Liganden wasserlöslich sind und dadurch nach wässriger Aufarbeitung das Metall in die wässrige Phase geht.

Die vorliegende Erfindung löst alle diese Aufgaben und liefert ein, attraktives Verfahren zur Kupplung von Alkoholaten zur Herstellung von Verbindungen der Formel (III) durch Umsetzung von Verbindungen der Formel (II) mit a) einem Alkoholat oder b) einem Alkohol R1-OH und einer Base

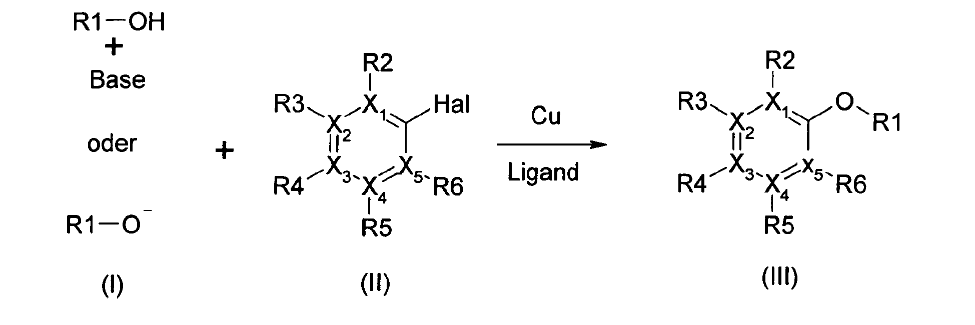

in Gegenwart eines Cu-enthaltenden Katalysators und einem Liganden,
wobei die Substituenten R1 bis R6, X₁ bis X₅ und Hal folgende Bedeutung haben:
R1 steht für einen Substituenten aus der Gruppe: Methyl, primäres, sekundäres oder tertiäres, cyclisches oder acyclisches C₁ bis C₁₂-Alkylradikal, substituiertes cyclisches oder acyclisches C₁ bis C₁₂Alkylradikal oder ein Phenyl-, substituiertes Phenyl-, Heteroaryl- oder substituiertes Heteroarylradikal;
R2-5 stehen für Substituenten aus der Gruppe: Wasserstoff, Methyl, primäres, sekundäres oder tertiäres, cyclisches oder acyclisches Alkylradikal, bei denen gegebenenfalls ein oder mehrere Wasserstoffatome durch Fluor oder Chlor ersetzt sind, z.B. CF₃, substituierte cyclische oder acyclische Alkylradikale, Alkoxy-, Dialkylamino-, Alkylamino-, Arylamino-, Diarylamino-, Phenyl-, substituierte Phenyl-, Heteroaryl-, substituierte Heteroaryl-, Alkylthio-, Arylthio-, Diarylphosphino-, Dialkylphosphino-, Alkylarylphosphino-, Dialkyl-, Arylalkyl- oder Diarylaminocarbonyl-, Monoalkyl- oder Monoarylaminocarbonyl-, CO₂⁻ , Alkyl- oder Aryloxycarbonyl-, Hydroxyalkyl-, Alkoxyalkyl-, Nitro-, Cyanoreste, oder zwei benachbarte Reste R2-5 bilden zusammen einem aromatischen, heteroaromatischen oder aliphatischen ankondensierten Ring; Hal steht für Chlor, Brom, lod, Alkylsulfonat oder Arylsulfonat,
X₁₋₅ bedeuten unabhängig voneinander entweder Kohlenstoff oder Stickstoff, oder jeweils zwei benachbarte über eine formale Doppelbindung verbundene XᵢRᵢ stehen gemeinsam für O (Furane), S (Thiophene), NRH oder NRᵢ (Pyrrole).

Dabei zeichnet sich das Verfahren dadurch aus, dass quantitative Umsetzungen erreicht werden können und die Reaktionszeiten typischerweise auf 8 bis 12 h reduziert sind. Gegenüber den klassischen Varianten der C,O-Kupplung weist diese neuartige Methode des weiteren den zusätzlichen Vorteil auf, dass die typischerweise als Liganden eingesetzten Oligoglykole und Polyglykole oder Polyamine keinerlei toxische Eigenschaften aufweisen und typischerweise nicht als Gefahrgut klassifiziert sind. Ein weiterer Vorteil des Verfahrens ist, dass sowohl die Liganden als auch die Kupferverbindungen ohne Probleme von den jeweiligen Kupplungsprodukten abzutrennen sind, da sie sich einerseits oftmals im Gegensatz zu den organischen Kupplungsprodukten sehr gut in Wasser lösen und andererseits oftmals hohe Siedepunkte aufweisen, die eine Kontamination des Kupplungsproduktes bei der destillativen Aufarbeitung verhindern.

Als Liganden werden acyclische und/oder cyclische Oligo- und Polyglykole, Oligo- und Polyamine, oder Oligo- oder Polyaminoglykole der allgemeinen Formel (IV) eingesetzt.

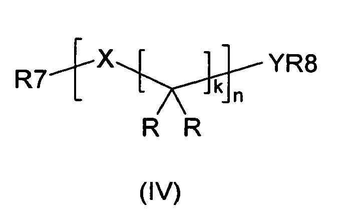

Dabei steht in Formel (IV) R7-8 für Substituenten aus der Gruppe {Wasserstoff, Methyl, primäre, sekundäre oder tertiäre, cyclische oder acyclische Alkylreste, bei denen gegebenenfalls ein oder mehrere Wasserstoffatome durch Fluor oder Chlor ersetzt sind, z.B. CF₃, substituierte cyclische oder acyclische Alkylgruppen}. Die Gruppen R7 und YR8 können dabei auch gemeinsam einen Ring bilden.
k steht für die Anzahl der [CR₂]-Einheiten und n für die Anzahl der [X[CR₂]ₖ]-Einheiten, wobei k eine ganze Zahl > 0 und n eine ganze Zahl > 1 ist.

Die Heteroatome X und Y stehen unabhängig voneinander für Sauerstoff oder Stickstoff (NH oder NRᵢ), wobei diese sowohl gleichzeitig beide Stickstoff (NH oder NRᵢ) sowie beide Sauerstoff oder getrennt voneinander Sauerstoff (NH oder NRᵢ) oder Stickstoff bedeuten können. Die Reste R in der Formel (IV) stehen für Substituenten der [CR₂]-Einheiten aus der Gruppe {Wasserstoff, Methyl, primäre, sekundäre oder tertiäre, cyclische oder acyclische Alkylreste, bei denen gegebenenfalls ein oder mehrere Wasserstoffatome durch Fluor oder Chlor ersetzt sind, z.B. CF₃, substituierte cyclische oder acyclische Alkylgruppen}. Die Substituenten R benachbarter [CR₂]-Einheiten können dabei auch zusammen eine Doppelbindung bilden oder Teil eines aromatischen Rings sein.

Die als Katalysator eingesetzte Spezies besteht aus Kupferpulver oder einer Kupferverbindung der Oxidationsstufe 0, +1 oder +2.

Bevorzugt werden Alkoholate eingesetzt, bei denen R₁ für Alkylreste steht wie Methyl, Ethyl, 2-Propyl oder tert-Butyl. Das Alkoholat R₁-O⁻ wird wahlweise, falls kommerziell erhältlich, zugekauft bzw. in einem vorgelagerten Schritt generiert oder in der Reaktionsmischung durch Gegenwart von Base während der Reaktion (nach-) gebildet. Dabei werden die entsprechenden Alkohole R₁-OH mit handelsüblichen Basen umgesetzt, beispielsweise mit Carbonaten, Hydriden, Hydroxiden oder Phosphaten.

Bevorzugte Halogenaromaten der Formel (II), die nach dem erfindungsgemäßen Verfahren umgesetzt werden können, sind z.B. Benzole, Pyridine, Pyrimidine, Pyrazine, Pyridazine, Furane, Thiophene, Pyrrole, beliebig N-substituierte Pyrrole oder Naphthaline. In Frage kommen beispielsweise 2-, 3- und 4-Brombenzotrifluorid, 2-, 3- und 4-lodbenzotrifluorid, 2-, 3-Bromthiophen, 2-, 3-, 4-Brompyridin, 3,5-Dimethylbrombenzol, 3,5-Dimethylchlorbenzol, 3,5-Dimethyliodbenzol.

Geeignete Lösungsmittel sind beispielsweise Tetrahydrofuran, Dioxan, Diethylether, Di-n-butylether, Diisopropylether oder Alkohole wie Methanol, Ethanol, 2-Propanol oder n-Butanol. Bevorzugt wird Methanol, Ethanol und 2-Propanol eingesetzt.

Die bevorzugten Reaktionstemperaturen liegen zwischen + 25°C und + 200°C, besonders bevorzugt sind Temperaturen zwischen 60 und 120°C.

Bevorzugt werden Verbindungen eingesetzt wie Kupfer(I)chlorid, Kupfer(I)bromid, Kupfer(I)iodid, Kupfer(I)oxid, Kupfer(II)acetylacetonat, Kupfer(II)bromid, Kupfer(II)iodid, besonders bevorzugt Kupfer(I)chlorid und Kupfer(I)bromid. Es kann in den meisten Fällen mit sehr geringen Mengen an Kupferkatalysator gearbeitet werden, wobei typische eingesetzte Kupfermengen zwischen 20 und 0.01 Mol%, besonders typische Mengen zwischen 5 und 0.05 Mol-% liegen.

Typische Beispiele für Liganden nach Formelschema (IV) sind Verbindungen wie Hexadecyloxypropanol, Octadecyloxyethanol, Hexadecyl-oxypropylmethylether, Octadecyloxyethyl-methylether, Polyethylenglykol 200, Polyethylenglykol 300, Polyethylenglykol 600, Polyethylenglykol 1000, Polyethylenglykol 20000, Polyethylenglykol 35000, Polyethylenglykoldimethylether 250, Polyethylenglykol-dimethylether 500, Polyethylenglykoldimethylether 2000, 1,4,10-Trioxa-7,13-diazacyclopentadecan, 2-(2-Aminoethoxy)ethanol, Tetraethylenpentamine sowie Kronenether wie 12-Krone-4, 18-Krone-6, Benzo-15-krone-5, Benzo-18-krone-6, Decyl-18-krone-6, Dibenzo-18-krone-6, N-Phenylaza-15-krone-6, wobei ein oder mehrere Verbindungen gemeinsam eingesetzt werden können. Es kann in den meisten Fällen mit sehr geringen Mengen an Liganden gearbeitet werden, wobei typische eingesetzte Ligandenmengen zwischen 40 und 0.02 Mol-%, besonders typische Mengen zwischen 10 und 0.1 Mol% liegen.

Die Aufarbeitungen geschehen im Allgemeinen wässrig, wobei entweder Wasser bzw. wässrige Mineralsäuren zudosiert werden oder die Reaktionsmischung auf Wasser bzw. wässrige Mineralsäuren dosiert wird. Zur Erzielung bester Ausbeuten wird hier jeweils der pH-Wert des zu isolierenden Produkts eingestellt, also für gewöhnlich ein leicht saurer, im Falle von Heterocyclen auch leicht alkalischer pH-Wert. Die Reaktionsmischungen werden im Allgemeinen nach der Wasserzugabe filtriert, um unlösliche Kupferverbindungen zu entfernen. Die Reaktionsprodukte werden beispielsweise durch Extraktion und Eindampfen der organischen Phasen gewonnen. Alternativ können auch aus der Hydrolysemischung die organischen Lösungsmittel abdestilliert und das dann ausfallende Produkt durch Filtration gewonnen werden.

Die Reinheiten der Produkte aus den erfindungsgemäßen Verfahren sind im allgemeinen hoch, für Spezialanwendungen (Pharmavorprodukte) kann allerdings noch ein weiterer Aufreinigungsschritt, beispielsweise durch Umkristallisation oder eine Feindestillation, durchgeführt werden. Die Ausbeuten an den Reaktionsprodukten betragen 60 bis 99 %, typische Ausbeuten sind insbesondere 85 bis 99 %.

Das erfindungsgemäße Verfahren eröffnet eine sehr ökonomische Methode der Transformation von aromatischen Halogeniden und Sulfonaten in die entsprechenden Alkoxy- oder Aryloxysubstituierten Verbindungen.

Das erfindungsgemäße Verfahren soll durch die nachfolgenden Beispiele erläutert werden, ohne die Erfindung darauf zu beschränken:

### Beispiel 1

Herstellung von 2-Methoxythiophen aus 2-Bromthiophen 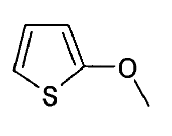 300 g 2-Bromthiophen (1,84 mol) werden in eine Mischung von 2,64 g Kupfer(I)bromid (CuBr, 1 Mol-%), 18,4 g Polyethylenglykol-dimethylether (PEG DME) 500 (2 Mol-%) und 660 g Natriummethanolat-Lösung in Methanol (30 %ig) eingebracht (Eduktkonzentration von 30,6 %) und auf 90°C erhitzt. Nach einem per GC bestimmten Umsatz von > 98 % (insgesamt 8 h) wird das Reaktionsgemisch auf 500 g Wasser gegeben. Anschließend wird über Decalite filtriert und die Mischung wird zwei Mal mit jeweils 150 g MTBE extrahiert. Aus den vereinigten organischen Phasen erhält man nach Vakuumfraktionierung 182 g 2-Methoxythiophen (1,59 mol, 86,4 %), GC-Reinheit > 99% a/a.

### Beispiel 2

Herstellung von 3-Methoxythiophen aus 3-Bromthiophen 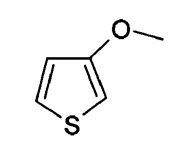 185 g 3-Bromthiophen (1.13 mol) werden in eine Mischung von 3.24 g Kupfer(I)bromid (CuBr, 2 Mol-%), 14.1 g PEG DME 250(5 Mol-%) und 407 g Natriummethanolat-Lösung in Methanol (30 %ig) eingebracht (Eduktkonzentration von 30.4 %) und auf 90°C erhitzt. Nach einem per GC bestimmten Umsatz von > 98 % (insgesamt 10 h) wird das Reaktionsgemisch auf 300 g Wasser gegeben. Anschließend über Decalite filtriert und die Mischung wird zwei Mal mit jeweils 120 g MTBE extrahiert. Aus den vereinigten organischen Phasen erhält man nach Vakuumfraktionierung 117,4 g 3-Methoxythiophen (1.03 mol, 91 %), GC-Reinheit > 99 % a/a.

### Beispiel 3

### Herstellung von 3,5-Bis(trifluormethyl)anisol aus 1-Brom-3,5-bis-(trifluormethyl)-benzol

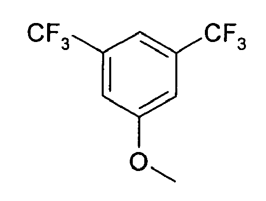

25 g an 1-Brom-3,5-bis-(trifluormethyl)benzol (85 mmol) werden in eine Mischung von 366 mg Kupfer(I)bromid (CuBr, 3 Mol-%), 1,.57 g 18-Krone-6 (7 Mol-%) und 92,3 g Natriummethanolat-Lösung in Methanol (30 %ig) eingebracht (Eduktkonzentration von 21 %) und auf 105°C erhitzt. Nach einem per GC bestimmten Umsatz von > 97% (insgesamt 22 h) wird das Reaktionsgemisch auf 175 g Wasser gegeben. Durch Dosierung von Salzsäure wird die Mischung auf pH 5-6 gebracht und anschließend wird über Decalite filtriert. Die Mischung wird zwei Mal mit jeweils 150 g Dichlormethan extrahiert. Aus den vereinigten organischen Phasen erhält man nach Vakuumfraktionierung 16,5 g 3,5-Bis(trifluormethyl)anisol (68 mmol, 79,4 %), GC-Reinheit > 98,5 % a/a.

### Beispiel 4

### Herstellung von 2-Ethoxy-3-(trifluormethyl)pyridin aus 2-Brom-3-(trifluormethyl)-pyridin

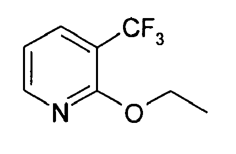

45 g an 2-Brom-3-(trifluormethyl)pyridin (0,2 mol) werden in eine Mischung von 574 mg Kupfer(I)bromid (CuBr, 2 Mol-%), 2 g (4 Mol-%) Polyethylenglykoldimethyl-ether 250 und 136 g Natriumethanolat-Lösung in Ethanol (20 %ig) eingebracht (Eduktkonzentration von 24,5 %) und auf 100°C erhitzt. Nach einem per GC bestimmten Umsatz von > 99 % (insgesamt 9 h) wird das Reaktionsgemisch auf 125 g Wasser gegeben. Durch Dosierung von Salzsäure wird die Mischung auf pH 9 gebracht und anschließend wird über Decalite filtriert. Die Mischung wird zwei Mal mit jeweils 135 g Toluol extrahiert. Aus den vereinigten organischen Phasen erhält man nach Vakuumfraktionierung 32.1 g 2-Ethoxy-3-(trifluormethyl)pyridin (0.17 mmol, 84 %), GC-Reinheit > 99 % a/a.

### Beispiel 5

### Herstellung von 2-Methoxy-5-methylpyridin aus 2-Brom-5-methylpyridin

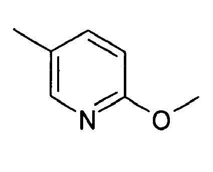

250 g an 2-Brom-5-methylpyridin (1,45 mol) werden in eine Mischung von 2,1 g Kupfer(I)bromid (CuBr, 1 Mol-%), 14.5 g (2 Mol-%) Polyethylenglykoldimethylether 500 und 457 g Natriummethanolat-Lösung in Methanol (30%ig) eingebracht (Eduktkonzentration von 34,5 %) und auf 90 °C erhitzt. Nach einem per GC bestimmten Umsatz von > 98.5 % (insgesamt 17 h) wird das Reaktionsgemisch auf 750 g Wasser gegeben. Durch Dosierung von Salzsäure wird die Mischung auf pH 9 gebracht und anschließend wird über Decalite filtriert. Die Mischung wird zwei Mal mit jeweils 350 g MTBE extrahiert. Aus den vereinigten organischen Phasen erhält man nach Vakuumfraktionierung 162,8 g 2-Methoxy-5-methylpyridin (1,32 mol, 91 %), GC-Reinheit > 98 % a/a.

### Beispiel 6

### Herstellung von 2-Ethoxy-3-methylthiophen aus 2-Brom-3-methylthiophen

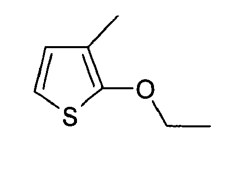

45 g an 2-Brom-3-methylthiophen (0.25 mol) werden in eine Mischung von 1,79 g Kupfer(I)bromid (CuBr, 5 Mol-%), 7.5 g (12 Mol-%) Polyethylenglykoldimethylether 250 und 255 g Natriumethanolat-Lösung in Ethanol (20 %ig) eingebracht (Eduktkonzentration von 14.5 %) und auf 110 °C erhitzt. Nach einem per GC bestimmten Umsatz von > 98.5 % (insgesamt 17 h) wird das Reaktionsgemisch auf 600 g Wasser gegeben. Anschließend wird über Decalite filtriert und die Mischung wird zwei Mal mit jeweils 350 g MTBE (Methyl-tert-butyl-ether) extrahiert. Aus den vereinigten organischen Phasen erhält man nach Vakuumfraktionierung 31,3 g 2-Ethoxy-3-methylthiophen (0.22 mol, 87 %), GC-Reinheit > 99% a/a.

## Patentansprüche

1. Verfahren zur Herstellung von Verbindungen der Formel (III) durch Umsetzung von Verbindungen der Formel (II) mit a.) einem Alkoholat oder b.) einem Alkohol R1-OH und einer Base 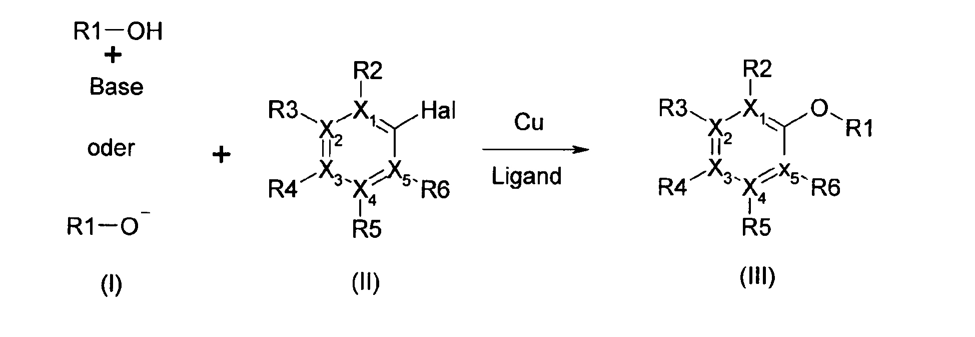 in Gegenwart eines Cu-enthaltenden Katalysators und einem Liganden,
wobei die Substituenten R1 bis R6, X₁ bis X₅ und Hal folgende Bedeutung haben:
R1 steht für einen Substituenten aus der Gruppe: Methyl, primäres, sekundäres oder tertiäres, cyclisches oder acyclisches C₁ bis C₁₂-Alkylradikal, substituiertes cyclisches oder acyclisches C₁ bis C₁₂-Alkylradikal oder ein Phenyl-, substituiertes Phenyl-, Heteroaryl- oder substituiertes Heteroarylradikal;
R2-5 stehen für Substituenten aus der Gruppe: Wasserstoff, Methyl, primäres, sekundäres oder tertiäres, cyclisches oder acyclisches Alkylradikal, bei denen gegebenenfalls ein oder mehrere Wasserstoffatome durch Fluor oder Chlor ersetzt sind, substituierte cyclische oder acyclische Alkylradikale, Alkoxy-, Dialkylamino-, Alkylamino-, Arylamino-, Diarylamino-, Phenyl-, substituierte Phenyl-, Heteroaryl-, substituierte Heteroaryl-, Alkylthio-, Arylthio-, Diarylphosphino-, Dialkylphosphino-, Alkylarylphosphino-, Dialkyl-, Arylalkyl- oder Diarylaminocarbonyl-, Monoalkyl- oder Monoarylaminocarbonyl-, CO₂⁻, Alkyl- oder Aryloxycarbonyl-, Hydroxyalkyl-, Alkoxyalkyl-, Nitro-, Cyanoreste, oder zwei benachbarte Reste R2-5 bilden zusammen einem aromatischen, heteroaromatischen oder aliphatischen ankondensierten Ring; Hal steht für Chlor, Brom, lod, Alkylsulfonat oder Arylsulfonat,
X₁₋₅ bedeuten unabhängig voneinander entweder Kohlenstoff oder Stickstoff, oder jeweils zwei benachbarte über eine formale Doppelbindung verbundene XᵢRᵢ , mit i=1-6, stehen gemeinsam für O, S, NRH oder NRᵢ .

2. Verfahren nach Anspruch 1, **dadurch gekennzeichnet, dass** als Liganden acyclische und/oder cyclische Oligo- und Polyglykole, Oligo- und Polyamine oder Oligo- und Polyaminoglykole der allgemeinen Formel (IV) eingesetzt werden, 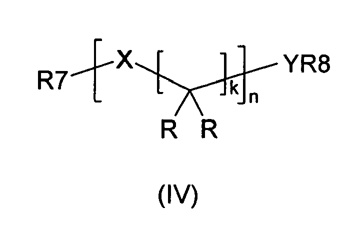 wobei R7-8 für Substituenten aus der folgenden Gruppe steht: Wasserstoff, Methyl, primäres, sekundäres oder tertiäres, cyclisches oder acyclisches Alkylradikal, bei denen gegebenenfalls ein oder mehrere Wasserstoffatome durch Fluor oder Chlor ersetzt sind, substituierte cyclische oder acyclische Alkylradikale oder die Gruppen R7 und YR8 gemeinsam einen Ring bilden;
k ist eine ganze Zahl > 0 und n eine ganze Zahl > 1;
X und Y stehen unabhängig voneinander für O, NH oder NRᵢ,
R steht für einen Substituenten aus der Gruppe: Wasserstoff, Methyl, primäres, sekundäres oder tertiäres, cyclisches oder acyclisches Alkylradikal, bei denen gegebenenfalls ein oder mehrere Wasserstoffatome durch Fluor oder Chlor ersetzt sind, oder substituierte cyclische oder acyclische Alkylradikale oder die Substituenten R benachbarter [CR₂]-Einheiten bilden zusammen eine Doppelbindung und stehen für ein Alkenylradikal oder sind Teil eines aromatischen Rings.

3. Verfahren nach Anspruch 1 und/oder 2, **dadurch gekennzeichnet, dass** als Ligand eine oder mehrere Verbindungen der folgenden Gruppe eingesetzt werden: Hexadecyloxypropanol, Octadecyloxyethanol, Hexadecyl-oxypropylmethylether, Octadecyloxyethyl-methylether, Polyethylenglykol 200, Polyethylenglykol 300, Polyethylenglykol 600, Polyethylenglykol 1000, Polyethylenglykol 20000, Polyethylenglykol 35000, Polyethylenglykoldimethylether 250, Polyethylenglykol-dimethylether 500, Polyethylenglykoldimethylether 2000, 1,4,10-Trioxa-7,13-diazacyclopentadecan, 2-(2-Aminoethoxy)ethanol, Tetraethylenpentamine, Kronenether.

4. Verfahren nach mindestens einem der vorhergehenden Ansprüche, **dadurch gekennzeichnet, dass** der Katalysator Kupfer in der Oxidationsstufe 0, +1 oder +2 enthält.

5. Verfahren nach Anspruch 4, **dadurch gekennzeichnet, dass** der Katalysator Kupfer(I)chlorid, Kupfer(I)bromid, Kupfer(I)iodid, Kupfer(I)oxid, Kupfer(II)acetylacetonat, Kupfer(II)bromid, Kupfer(II)iodid oder Kupferpulver enthält.

6. Verfahren nach mindestens einem der vorhergehenden Ansprüche, **dadurch gekennzeichnet, dass** der Katalysator Kupfer im Bereich von 20 bis 0.01 Mol%, enthält.

7. Verfahren nach mindestens einem der vorhergehenden Ansprüche, **dadurch gekennzeichnet, dass** der Ligand in Mengen von 40 bis 0.02 mol eingesetzt wird.

8. Verfahren nach mindestens einem der vorhergehenden Ansprüche, **dadurch gekennzeichnet, dass** als Ausgangsverbindungen der Formel (II) Benzole, Pyridine, Pyrimidine, Pyrazine, Pyridazine, Furane, Thiophene oder gegebenenfalls substituierte Pyrrole oder Naphthaline eingesetzt werden.

9. Verfahren nach mindestens einem der vorhergehenden Ansprüche, **dadurch gekennzeichnet, dass** das Verfahren in einem Lösungsmittel aus der Gruppe: Tetrahydrofuran, Dioxan, Diethylether, Di-n-butylether, Diisopropylether, Methanol, Ethanol, 2-Propanol oder n-Butanol durchgeführt wird.

10. Verfahren nach mindestens einem der vorhergehenden Ansprüche, **dadurch gekennzeichnet, dass** das Verfahren bei einer Temperatur im Bereich von + 25°C bis +200°C durchgeführt wird.
